# EUROPEAN PATENT SPECIFICATION

(11) **EP 4 463 057 B1**
(45) Date of publication and mention of the grant of the patent: **09.12.2026**
(21) Application number: 22701160.8
(22) Date of filing: 10.01.2022
(51) Int. Cl.: A61B 5/00, A61B 5/11, A61B 5/113, A61B 5/0507, G01S 13/02

(54) **METHOD FOR DETERMINING A POSTURE OF A HUMAN BEING**
VERFAHREN ZUR BESTIMMUNG EINER HALTUNG EINES MENSCHEN
MÉTHODE DE DÉTERMINATION D'UNE POSTURE D'UN ÊTRE HUMAIN

(43) Date of publication of application: 20.11.2024
(73) Proprietor: Qumea AG, 4500 Solothurn (CH)
(72) Inventor: GYGER, Cyrill, 4500 Solothurn (CH); GERSHONI, Ido, 3672 Oberdiessbach (CH); REBSAMEN, Philipp, 3210 Kerzers (CH); REBER, Jonas, 3308 Grafenried (CH)
(74) Representative: Keller Schneider Patent- und Markenanwälte AG
(86) International application number: PCT/EP2022/050346
(87) International publication number: WO 2023/131422

(56) References cited:
- EP-B1- 3 286 577
- WO-A1-2021/130690
- CN-A- 112 346 055
- US-A1- 2020 378 758
- CUI HAN ET AL: "Real-Time Short-Range Human Posture Estimation Using mmWave Radars and Neural Networks", IEEE SENSORS JOURNAL, IEEE, USA, vol. 22, no. 1, 15 November 2021 (2021-11-15), pages 535 - 543, XP011896355, ISSN: 1530-437X, [retrieved on 20211228], DOI: 10.1109/JSEN.2021.3127937
- LI GUANGZHENG ET AL: "Capturing Human Pose Using mmWave Radar", 2020 IEEE INTERNATIONAL CONFERENCE ON PERVASIVE COMPUTING AND COMMUNICATIONS WORKSHOPS (PERCOM WORKSHOPS), IEEE, 23 March 2020 (2020-03-23), pages 1 - 6, XP033806177, DOI: 10.1109/PERCOMWORKSHOPS48775.2020.9156151
- WANG QING WANGQINGYJ@CHINAMOBILE COM ET AL: "CLGNet: A New Network for Human Pose Estimation using Commodity Millimeter Wave Radar", 2020 THE 4TH INTERNATIONAL CONFERENCE ON BIG DATA RESEARCH (ICBDR'20), ACMPUB27, NEW YORK, NY, USA, 24 December 2020 (2020-12-24), pages 1 - 5, XP058586858, ISBN: 978-1-4503-8896-2, DOI: 10.1145/3446132.3446421

## Description

### Technical Field

The invention relates to a method for determining a posture of a human being based on at least two dimensional radar measurements, in particular for fall prevention. The invention further relates to a system for determining a posture of a human being.

### Background Art

Falling, especially of elderly people, is a common cause for injuries and other medical complications, whether it happened at home or in an institution. Especially for institutions (e.g. hospital, nursing home etc.) falls also have significant financial consequences, even if the person was completely uninjured, due to bureaucracy and office work as well as medical examinations.

The common existing methods for preventing falls mainly involve restricting a person's movements, for example by using a bed or a wheelchair bar. While those methods are effective, they can only be used with patients who have constant access to caring staff, e.g. while being cared for in an institution. Nevertheless, in most situations those methods are unfavorable, as they limit the person's freedom to move which can cause stress and slow down healing processes. In private environments they are in most cases impracticable.

Most falls occur when a person is trying to stand up (from a bed, chair, wheelchair etc.) or changing his or her location (e.g. from a rolling-walker to table, to toilet etc.). If a helper (e. g. a caregiver or family member) is informed early enough about the person's intention, they can assist the person with those actions. This can be easily done for example if the person is requesting help by himself or herself. Unfortunately, for elderly people in a confused state, in delirium or for people who suffer from cognitive dementia, this is not a reliable solution.

Accordingly, automated systems for the prevention of falls have been proposed. For instance, WO 2021/050966 A1 (Resmed) describes a system including a sensor configured to generate data associated with movements of a resident. The generated data is analyzed in order to determine a likelihood for a fall event to occur for the resident. If the likelihood satisfies a threshold, an operation of an electronic device is modified. Different sensor modalities are proposed to identify various aspects of the resident's behaviour as well as physiological data. However, no specific way of gathering and processing data is described.

The article H. Cui, N. Dahnoun, "Real-Time Short-Range Human Posture Estimation Using mmWave Radars and Neural Networks", IEEE Sensors Journal, Vol. 22, No. 1, January 1, 2022 relates to a human posture estimation system using two mmWave radars and a neural network model. It estimates the posture by localizing the key joints.

The article G. Li, Z. Zhang, H. Yang, J. Pan, D. Chen, J. Zhang, "Capturing Human Pose Using mmWave Radar", 2020 IEEE International Conference on Pervasive Computing and Communications (PERCOM) Workshops, IEEE, 23 March 2020 relates to human pose estimation as well. The described system uses two radar data to generate heatmaps and then adopts a convolutional neural network to transform these heatmaps into human pose.

The article Q. Wang, K. Wang, W. Chen, "CLGNet: A New Network for Human Pose Estimation using Commodity Millimeter Wave Radar", 2020 The 4th International Conference on Big Data Research (ICBDR'20), ACMPUB27, New York, NY, USA, 24 December 2020 relates to human pose estimation based on commodity millimeter wave radar. The mm-range radar sensors provide a 3D point cloud. This is clustered, and 30 consecutive clustered frames are aggregated for neural network analysis to obtain the pose estimation.

### Summary of the invention

It is therefore the object of the invention to create a method and a system for determining a posture of a human being pertaining to the technical field initially mentioned, that allow for a reliable determination based on a limited number of sensor modalities.

The solution of the invention is specified by the features of claim 1. According to the invention the method comprises the following steps:
a) obtaining measurement data from at least two dimensional radar measurements;
b) processing the measurement data to obtain at least two dimensional point cloud data, in particular three dimensional point cloud data, a position and local data being assigned to each of the points of the point cloud data, the local data comprising velocity vectors of the points, wherein the point cloud data is received at regular time intervals corresponding to frames;
c) clustering the point cloud data to obtain clustered data representing the human being by a plurality of clusters, wherein a cluster is a group of points of the point cloud which belongs to the same moving object;
d) tracking the clusters from frame to frame over time to define a plurality of objects, wherein
   - it is calculated for every cluster in every frame the probabilities that it relates to the same detected object as a certain cluster in the previous frame,
   - for the clustering of body parts the velocity vectors of the points of the point cloud are taken into account,
   - for defining the objects data obtained from tracking the clusters, covering a time interval, is analyzed to separate static from dynamic objects;
e) identify body parts of the human being and positional relationships among them based on the plurality of objects, wherein the identified body parts comprise the head and/or limbs; and
f) analyze the positional relationships to determine the posture of the human being.

Analogously, a system for determining a posture of a human being comprises:
a) a radar system for generating measurement data from at least two dimensional radar measurements from a space accommodating the human being;
b) a first processor adapted to process the measurement data to obtain at least two dimensional point cloud data, in particular three dimensional point cloud data, a position and local data being assigned to each of the points of the point cloud data, the local data comprising velocity vectors of the points, wherein the point cloud data is received at regular time intervals corresponding to frame;
c) a second processor adapted to cluster the point cloud data to obtain clustered data representing the human by a plurality of clusters, wherein a cluster is a group of points of the point cloud which belongs to the same moving object;
d) a tracker adapted to track the clusters from frame to frame over time to define objects, the tracker being adapted to:
   - calculate for every cluster in every frame the probabilities that it relates to the same detected object as a certain cluster in the previous frame, taking into account the velocity vectors of the points of the point cloud for the clustering of body parts,
   - analyse data obtained from tracking the clusters, covering a time interval, to separate static from dynamic objects, for defining the objects;
e) an identifier adapted to identify body parts of the human being and positional relationships among them based on the plurality of objects, wherein the identified body parts comprise the head and/or limbs; and
f) an analyzer adapted to analyze the positional relationships to determine the posture of the human being.

Preferably, the radar system comprises units featuring senders and receivers arranged in a common unit. The number of senders and receivers is chosen depending on the employed electromagnetic waves and the desired information.

The units are arranged in a distance from the patient. In particular, the distance of the units from the patients is at least 1 m, preferably at least 2 m. Accordingly, they do not bother the patient and there is no need to attach electronics to the patient (in contrast to e. g. wearables). Preferably, the units are independent from patient-specific installations such as (hospital) beds or chairs. Most preferably, the units are fixedly arranged in the room, e. g. attached to the ceiling or a wall. It is advantageous if the monitoring volume of the installation covers several or all potential patient sites, e. g. spaces where beds are placed in a room. This allows for a very flexible use and dispenses with physical rearrangement of the monitoring installation. Furthermore, being able to monitor several patients with a single device reduces infrastructure expenses.

Depending on the chosen frequency, energy and material properties, radio waves can penetrate objects such as bed sheets and thus allow for precise monitoring of human beings even if they are e. g. lying in a bed. At the same time it is not required to generate a representational image of the patient in order to be able to obtain the desired information, thus safeguarding the patient's privacy.

In particular, the radar measurements are obtained from a wideband radar system, i. e. a radar system having a bandwidth of at least 100 MHz. In a preferred embodiment, the wideband radar system is an ultra-wideband (UWB) radar system. Such systems have a bandwidth exceeding the lesser of 500 MHz or 20% of the arithmetic center frequency.

Preferably, the center frequency of the wideband or ultra-wideband radar system is in the range of 2 - 75 GHz. Advantageously, the range resolution of the radar system is 5 mm or better.

Wideband or ultra-wideband radar systems as well as center frequencies in the given range allow for high resolution, sufficient penetration of objects such as bed sheets and sufficient reflection from the body of the patient to be monitored.

In a preferred embodiment, the wideband radar system is a MIMO (multiple-input multiple-output) radar system, featuring a number of transmitting antennas sending different transmitting signals (in particular orthogonal or intermittent signals) as well as a number of receiving antennas receiving signals from different transmitting antennas. Signals relating to different transmitting antennas may be extracted from the received signals using matched filters. MIMO radar systems may be built compactly, and they offer improved spatial resolution and Doppler resolution compared to 1D systems.

In particular, for spatial resolution, direction of arrival (DoA) values (alternative term: angle of arrival AoA) may be obtained from the phase difference and/or from the time difference of signals received from different receiving antennas. Knowing the DoA values (with respect to a certain reference point) as well as the distances from the individual antennas the 2- or 3-dimensional position of a target is defined. In principle, the signals from any number of antennas being adapted to receive signals from the search area may be utilized to obtain position information. With respect to stationary or slowly moving objects, multiple signals from the same antenna, taken during a certain time interval, may be utilized.

In order to be able to obtain the direction of arrival, the receiving positions of the at least two receiving antennas are arranged in a certain distance from each other. In particular, the at least two receiving antennas are constituted by at least two physical antennas. Alternatively, the at least two receiving antennas are constituted by a single antenna which is moved between different receiving positions by a suitable mechanism, e. g. a revolving mechanism. Accordingly, a moving receiving antenna constitutes "at least two receiving antennas" in the sense of the present invention.

A density-based clustering algorithm is employed, which locates high-density areas in the n-dimensional space. Accordingly, a cluster is a group of points of the point cloud which belong to the same moving object, regardless of its shape. Algorithms for clustering point cloud data are readily available. In preferred embodiments, an unsupervised statistical learning algorithm is employed. In general, the resulting clusters of the plurality of clusters can have an arbitrary form and size, depending on the detected movements and their distribution. In order to further process the clustered data each of the plurality of clusters may be represented by a model, in particular by a geometric shape fitted to the points assigned to the respective clusters. In the context of three dimensional point clouds, suitable shapes may comprise ellipsoids, cuboids, circular cylinders, etc. and the model may be characterized by parameters relating to the dimensions of the respective shape (e. g. length, width, height), the position of the shape (e. g. its central point, indicated by three Cartesian coordinates) and an orientation in space (indicated e. g. by three Euler angles).

For defining the plurality of objects, the plurality of clusters in the clustered data are tracked from frame to frame. The tracking algorithm calculates for every cluster in every frame the probabilities that it relates to the same detected object (part) as a certain cluster in the previous frame. The probability calculation takes into account different characteristics of the clusters, e. g. the current position, path so far (direction and velocity) and its points' reflection characteristics. Each tracked cluster is then identified as an object and is given a unique identifier.

An object may relate to the human being and/or to body parts thereof. Basically, for clustering entire human beings, the (three-dimensional) positions of the points of the point cloud may be used, whereas for the clustering of specific body parts further information is taken into account, in particular the velocity vectors of the points of the point cloud. Accordingly, depending on the way of clustering the defined objects will relate to the entire human beings or to their body parts. In order to obtain comprehensive information, both types of clusters and objects may be established and further processed. Preferably, in this case each of the "body part objects" is assigned to one of the "human objects".

The objects are characterized by a number of properties, in particular by one or several of the following:
- The size and form of the object, as well as its position and orientation. For this purpose, a model for the object may be used that is based on the same principles as the model for the cluster described above.
- The density and distribution of points in the cluster(s) of the respective object. If the detection of the points is based on movement, a higher density of points means that more movement is being generated in the respective region. Therefore, changes in the distribution indicate the amplitude of movements in certain areas of the cluster, i.e. of different body parts.
- Velocity and direction of movement for each point in the cluster(s) of the respective object. They give an indication of the changes in movements in a higher resolution.
- Statistical information, collected over time, e. g. the movement pattern of the object and/or parts thereof, the amplitudes and frequencies of these movements.
- Environment-related information, e. g. a height above a floor, distances from other objects, etc.

The tracking of the clusters and definition of objects based on the tracked clusters allows for the reliable identification and definition of objects despite the fact that in many intervals for many parts of the body the detected movements will be little or even missing.

Basically, relevant information collected over time is accumulated and employed for the determination of the posture as well as transitions between different postures.

Every fall is preceded by a series of movements which can indicate that a certain person is about to fall. For a standing person it can be a loss of balance or stumbling. For a lying or sitting person those are the movements which indicate standing up or preparation for standing up. The inventive method and system allows for detecting those movements' patterns and for providing an early alert so that a helper could either assist with the action or calm down a confused person. This early alert can drastically reduce the risk of falls without affecting a person's freedom. Furthermore, such a method and system have the potential to support better care and get assistance for confused patients when they need it, even if they are cognitively unable to request it by themselves.

Accordingly, the inventive method may further comprise the step of triggering an alert, e.g. to a caregiver, when predetermined alert conditions are met. The conditions may include a fall probability, determined from the determined posture of the human being and/or determined posture transitions. Further input data may be considered, e. g. relating to the recent history of determined postures or transitions or to previous near-fall or fall events of the human being.

Preferably, the local data assigned to each of the points of the point cloud data comprises velocity information, in particular vectorial velocity information.

The vectorial velocity information includes (scalar) velocity information as well as the direction of the movement. Preferably, the velocity information is obtained from Doppler information within the measurement data.

Preferably, before feeding the point cloud data to the clustering algorithm, the data from each point of the point cloud data is converted into a feature-vector. To achieve the most accurate grouping, each point is converted into a high-dimensional vector which contains not only the position of the point but further information.

Accordingly, it is preferred that the local data assigned to each of the points of the point cloud data comprises a reflection magnitude. Taking into account the reflection characteristics allows for better distinguishing between points that are in a close positional relationship but relate to different objects.

Preferably, the local data assigned to each of the points of the point cloud data comprises a signal-to-noise ratio of an underlying radar measurement (or a plurality of underlying radar measurements) and/or a measure for a confidence of the position and/or the local data.

Taking into account such data allows for further improving the results of the clustering, the definition of objects and/or the further processing. Corresponding data is usually readily available from radar systems. Depending on the signal-to-noise ratio the points may be considered with different weights and/or points based on low-quality measurements may be disregarded at all. The confidence measure may be obtained from the matrix inversion process employed for the determination of the points of the point cloud. Again, the confidence measure may be used to set weights and/or to exclude certain points from further processing.

**In** order to further improve the results, the clustering algorithm may also be configured to eliminate noise by detecting not-clustered points ("outliers") and irregular groups.

Advantageously, the objects are classified to identify non-human objects and in that objects representing non-human objects are excluded from the identification of the body parts of the human being and the positional relationships among them.

Preferably, the classification is done by a machine-learning algorithm which was trained on a dedicated high-dimensional feature vector extracted from every object. **In** particular, objects which are classified as non-humans are being removed and their points' data is not used for the rest of the algorithm. Nevertheless, it may be used for further processing steps that are not directly related with the determination of the posture of the human being.

For defining the objects data obtained from tracking the clusters, covering a time interval, is analyzed to separate static from dynamic objects. In particular, this allows for separating static patients who are e. g. lying in a bed from dynamic caregivers who are walking around in the room and/or enter or leave the room.

Static objects located in predefined zones may be identified as patients. The predefined zones may e. g. relate to the location of beds, chairs or similar facilities. The amount of time in which the object's data is being collected for this assessment may be determined according to the amount of movement of an object and the distribution of the related points in space as well as in time.

In a preferred embodiment, an activity level is determined from the point cloud data and an overall shape defined by the points of the plurality of clusters representing the human being is analyzed to determine the posture if the activity level exceeds a threshold.

Movements such as sitting up, standing up or walking involve considerably more activity than e. g. change of posture of a lying person or even micro-movements due to breathing or heartbeat. Accordingly, the detection of such movements does not require the identification of individual body parts and their positional relationship but may be identified from an overall shape of a point cloud representing the entire human being. In such cases, due to the points of the point cloud moving basically as a whole the overall shape including its position and movement vector is easily obtained from the point cloud data. The identification of the related basic postures "lying", "sitting" and "standing" may be obtained e. g. using a machine-learning algorithm, trained on labeled samples.

The threshold for switching to this modality may be set during a calibration phase of the system. The activity level may be obtained from the movement information within the measurement or point cloud data and/or based on criteria relating to the position of points assigned to a monitored human being, e. g. a certain number of points outside a predefined (bed) zone or similar.

In order to identify posture transitions, the inventive method preferably comprises the further steps of:
- mapping further points of the point cloud data, obtained from further measurements after determining the posture, to the determined posture; and
- identifying points of the mapped further points lying outside of the determined posture in order to identify a posture transition.

Similarly, the tracker's positions from each object are compared from frame to frame to detect changes in the location in the room without posture change (e.g. moving with a wheelchair, rolling from a floor-bed etc.)
Preferably, a risk for the human being experiencing a fall incident is determined based on the posture and/or the posture transition and an alarm condition is set for the human being if the risk exceeds a threshold.

This allows for the prediction of imminent fall incidents. The setting of the alarm condition may be linked to triggering an alarm message, e. g. to a caregiver. One of the most important cases related to an increased fall risk is a lying patient standing up, which is usually preceded by sitting up. Therefore, preferably, a raised probability of a sitting up / standing up event is detected from the postures and posture transitions.

In particular, the determination of the risk is done using one of the following methods:
- Direct analysis: From Health-Care research papers and other knowledge bases, known situations which indicates that a person is about to stand-up or change his location are identified. Each of these situations is manually being analyzed to determine what movement-patters it generates. The system is then either programmed or trained to identify those (and similar) patterns. After identified, each situation is mapped to its stand-up probability, as interpreted from the information obtained from the knowledge bases.
- Statistical analysis: For this method data recorded by the system is used, in which every occurrence of a person standing-up or changing his or her location is marked (labeled). Those recordings are fed into a learning system, which then directly recognizes which patterns precede stand-up and location-change occurrences. Given enough recordings, the system calculates for each movement-pattern the probability that it will lead to a person standing-up or changing his location. After the system has processed all those recordings ("training"), it can continuously analyze data from movement-patterns and map them to the learned probabilities. If a pattern is not recognized, the transition probability "0" may be assumed and a transition is detected only after recognizing a corresponding pattern. Alternatively, a precautionary alarm may is triggered.

Preferably, the system offers an interface, in which a caregiver can chose for which stand-up-probability value he or she would like to be informed. In particular, this choice can be made patient-specific. The possible values to choose from are in generally discrete, but theoretically the system could offer a continuous model. For example, for a patient with very high risk of falling or for a patient who is standing up very quickly, a lower probability value should be chosen (i.e. inform even if the probability, that the patient will stand up soon, is low). This will make sure that the caregiver can arrive at the patient before he or she falls. For very slow patients or for patients who are able to stand up safely by themselves in most cases, a higher probability value can be chosen.

Advantageously, the alarm condition for the human being is reset if it is determined that the human being is approached by a dynamic object representing a caregiver.

The system is designed to suppress alerts when patients are being cared for (e.g. no "patient standing up" alert should be generated when a patient is being assisted in getting up and existing alarm conditions should be reset). Preferably, the path of every dynamic object is monitored. If a dynamic object is approaching a patient, it will be marked as "being cared for" and no alerts will be generated for that patient until the caregiver has left the patient.

In preferred embodiments, a range Doppler map is obtained from the at least two dimensional radar measurements and information on obtained clusters, defined objects and/or identified body parts is used to identify regions of interest in the range Doppler map, the regions of interest of the range Doppler map being analyzed in order to do at least one of the following:
- identify valid radar measurements to be considered for obtaining the at least two dimensional point cloud data;
- select radar measurements to be considered for extracting vital sign information; and
- clustering micro Doppler radar measurements in one of the regions of interest or starting from a region of interest.

In a preferred embodiment, range Doppler maps are generated from the received radio waves. Range Doppler maps relate the distance of targets from a receiving antenna to their relative velocity away from or towards the receiving antenna. Accordingly, signatures relating to body movements may be identified based on range Doppler data.

Now, the information obtained from the clustering, object definition and/or body part identification can be employed to process the more fundamental range Doppler data, which allows for accessing the comprehensive and detailed radar data (low-level data) in a targeted manner. The filtering of the radar measurements may be directed to the identification of body parts and filter out e. g. radar data relating to events involving speeds outside a certain speed range or accelerations outside a certain acceleration range as this data might be due to artefacts or relate to irrelevant events. Similarly, radar measurements that are relevant for the extraction of vital sign information may be identified at an early stage, filtering out all the rest, thus greatly simplifying the further analysis. The clustering of micro Doppler radar measurements in certain regions facilitates the classification of specific movement patterns or the extraction of movement anomalies.

In a preferred variant, the inventive method comprises the further steps of
- identifying an object that represents a chest of a human being, based on characteristic features relating to breathing movements and/or heartbeat; and
- determining a positional relationship of other objects to the identified chest object to identify the body parts of the human being and the positional relationships among them.

This improves the correct determination of the body parts. Preferably, identification of the chest region is repeated in regular intervals and/or if a positional change of the patient is detected using methods as described above.

Preferably, a movement activity level is determined from an amplitude and/or phase of the measurement data, the activity level is classified into at least one high activity level class and into at least one low activity level class and the amplitude and/or phase of the measurement data relating to an activity level classified in the at least one low activity level class is analyzed to identify the position of the chest region.

Without superimposed (gross) body movements, this identification of the breathing movement is substantially facilitated and improved.

Different methods for determining the activity level are available. In first embodiments of the inventive method, the movement activity level is determined based on solely the amplitude of the radar measurements. In particular, the amplitude of the received electromagnetic waves is compared to the amplitude of the emitted waves. This comparison yields information on the absorption and reflection of the electromagnetic waves. It may thus be an indication of the absorption and reflection properties of objects in the monitored space.

In second embodiments of the inventive method, the movement activity level is determined based solely on phase information of the radar measurements. In particular, a change in phase between the emitted and received waves is compared. This yields in particular information related to movements of the objects in the monitored space.

In third embodiments of the inventive method, the movement activity level is determined based on the amplitude as well as on the phase of the radar measurements.

The at least one low activity level class may relate to an inactive human being showing no substantial gross motor movements but only micro-movements relating to body functions such as breathing, heartbeat and digestion. The at least one high activity level class may relate to a human being showing gross motor movements, including e. g. movements of the limbs, e. g. movements that are related to a repositioning of the person's body.

More than two activity level classes may be defined, wherein one or several of the classes are considered to be low activity level classes, considered to obtain the information on the body position of the human being.

In particular, the information on the body position of the human being is obtained exclusively based on values relating to one low activity level class or several low activity level classes, without considering values relating to one high level activity class or several high level activity classes. Preferably, the classes are defined such that all gross motor movements that may relate to a repositioning lead to classifying the related activity level into a high activity level class, such that the information on the body position of the human being is exclusively obtained from values relating to phases without repositioning movements.

Subsequently, each object is preferably classified according to its geometrical shape into a torso candidate class or a non-torso candidate class. The criteria for classification may relate to the shape itself, to absolute dimensions and/or to relative measures such as aspect ratios. Positional relationships between the objects may be taken into account as well. Classification may be effected by supervised or unsupervised learning approaches or by conventional techniques, based on metrics such as the ones mentioned above, obtained from the objects and/or underlying data.

Preferably, for each of the objects a single value is calculated for all the points of the point cloud(s) assigned to the respective object, collected during a first sample period, the single value representing an activity level in the respective object.

In particular, the single value is calculated from a number of points collected and absolute velocities of each of the points.

These single values are collected over a second sample period. In particular, the second sample period covers a number of breathing cycles, e. g. 5-6 breathing cycles, corresponding to about 1 minute.

In a subsequent step, a frequency analysis is preferably performed over the collected single values and each of the objects is classified into a chest candidate class or a non-chest candidate class, based on the result of the frequency analysis.

The frequency analysis may be based on a Fourier transform (DFT/FFT), Wavelet Transform or Z-transform. Breathing movements lead to frequencies of about 0.07-0.17 Hz. The stronger such frequencies are present in the frequency domain, the higher the probability is that the respective object relates to the person's chest.

In most cases, in the frequency domain one of the objects will show clear indications of respiratory movement, while the other objects will be free of such signs. If none of the objects is classified into the chest candidate class, the analysis may be repeated. If the result persists, an alarm may be triggered. If more than one object is classified into the chest candidate class, the analysis may be repeated as well. In addition it may be checked whether the two or more objects in the chest candidate class are actually part of a superordinate "chest" object. In this case, the objects may be fused together into a single object or one of the candidate objects may be chosen to represent the patient's chest.

Instead or in addition to the results of the frequency analysis, information on the geometry of the clusters may be taken into account for identifying the chest.

Alternatively, not only the single values calculated for the objects are taken into account for the frequency and breathing analysis, but the information is obtained directly from range Doppler information relating to the respective object. In particular, the breathing rate as well as the heart rate may be obtained from phase information of the Doppler signals.

Instead of or in addition to the breathing movements, movements relating to the person's heartbeat may be identified and respective information may be processed to identify the person's chest. As a matter of course, the relevant frequencies will be higher, e. g. in the range of 0.8-1.4 Hz. Similarly, the first and second sample periods may be chosen differently, e. g. 1-2 s and 5-10 s, respectively.

Preferably, information relating to the chest region and information relating to other regions of the patient and a positional relationship between the chest region and the other regions are processed to obtain the information on the body position of the patient.

In particular, the chest area is defined as the convex hull around all the movement points in the chest cluster plus a small margin. Movement points from this "chest area" are now being collected separately from the rest of the movement-points from that person, deemed to relate to extremities. The positional relationship of the extremities and the chest/torso provides valuable information relating to the person's body position.

Other advantageous embodiments and combinations of features come out from the detailed description below and the entirety of the claims.

### Brief description of the drawings

The drawings used to explain the embodiments show:
- Fig. 1: A schematic representation of a hospital room with an embodiment of an inventive radar device installed; and
- Fig. 2: a schematic block diagram of the radar device.

In the figures, the same components are given the same reference symbols.

### Preferred embodiments

In the following, an embodiment of the invention is described, the described example relating to the monitoring of patients in a hospital environment. The general principles may be applied to other fields of application.

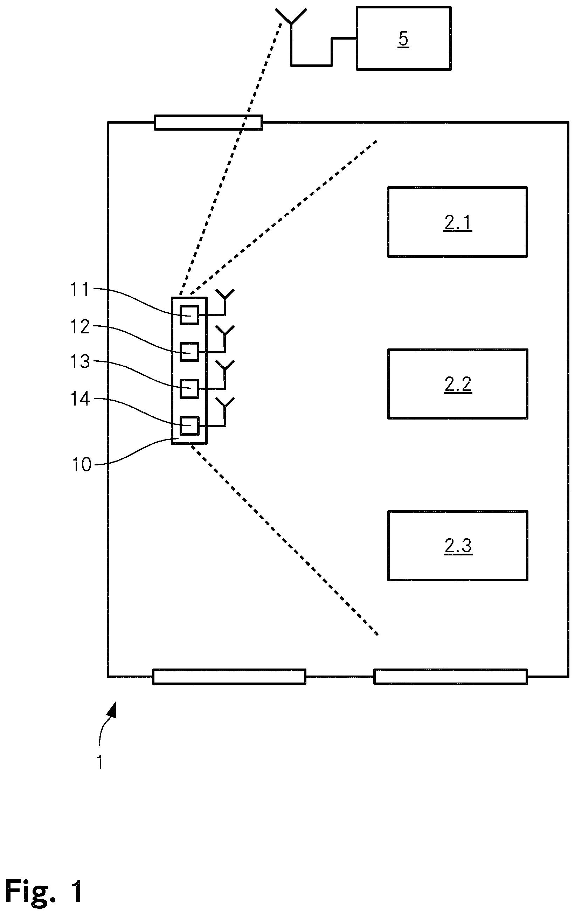

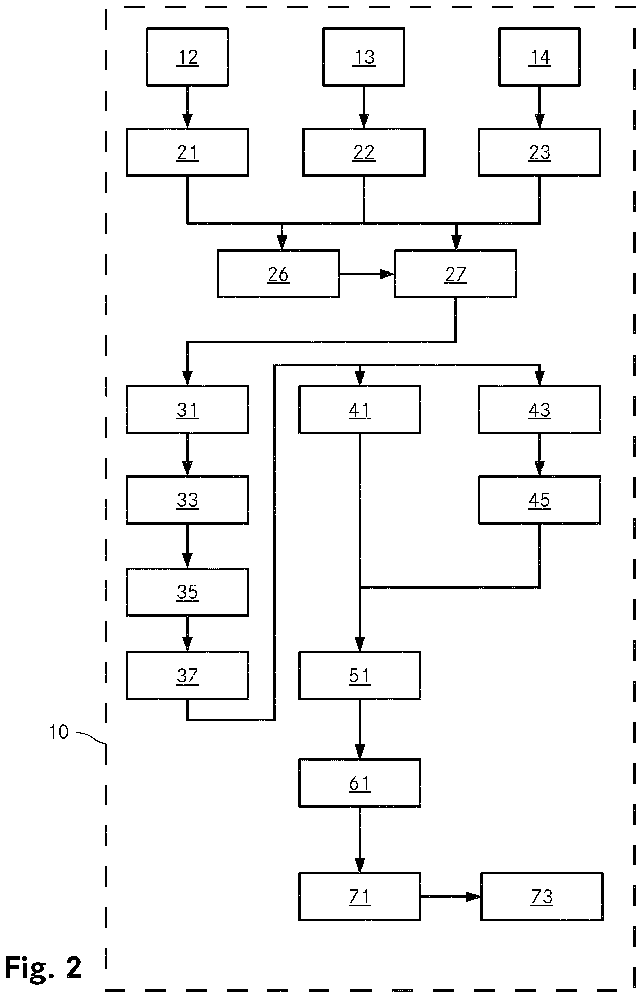

The Figure 1 is a schematic representation of a hospital room with an embodiment of the inventive system installed. Three beds 2.1, 2.2, 2.3 are placed in hospital room 1, along one of the side walls. The radar device 10 is attached to the ceiling of the room 1, its vertical distance to the support surfaces of the beds 2.1...3 is about 2-3 m. The radar device 10 comprises a transmitter 11 and three receivers 12, 13, 14. The transmitter 11 and the three receivers 12, 13, 14 are arranged within a housing of the radar device 10 in a certain distance from each other such that differences with respect to incoming signal phase are caused. Accordingly, the housing provides a certain linear extension. The radar device 10 is described in more detail below, with respect to Figure 2.

The basically cone-shaped monitoring volume covered by the radar device 10, depicted by dashed lines, covers all the three beds 2.1...3. In addition to the transmitter 11 and the receivers 12, 13, 14, the radar system 10 comprises a power supply, acquisition and processing electronics as well as an interface for exporting radar data to further devices; the radar device 10 is connected to a central server 5 by means of a wireless communication link.

The radar device 10 may provide data on different processing levels, i. e. raw radar data, angle data, range Doppler maps and/or even lists of persons with assigned positions, activity and vital sign information. Depending on the level of detail of the processed information, more or less further processing steps are carried out by the server 5. The server 5 is also used for displaying and/or storing the obtained data. It may further be used for configuring the radar system.

The Figure 2 is a schematic block diagram of a radar device according to the invention. The device includes a MIMO UWB radar system 10 comprising a sender 11 and three receivers 12, 13, 14. The centre frequency of the radar system is between 2 and 75 GHz.

The three receivers 12, 13, 14 provide radar data to 1-dimensional range Doppler modules 21, 22, 23 of the radar device 10. These modules deliver range Doppler data to a computing module 26 for calculating the angles of arrival based on the phase differences between the signals of the three receivers 12, 13, 14. Furthermore, a 3-dimensional Doppler map is generated by a further computing module 27 based on the range Doppler data as well as on the angles of arrival.

Basically, the range Doppler maps may be generated by the range Doppler modules 21, 22, 23, based on the raw radar data of several transceivers by the following steps:
1. The radar data is received by the processor from the transceivers. The data is organized in a matrix, where the columns correspond to separate, consecutive chirps. The lines of a given column represent the samples of the given chirp.
2. A matrix of a symmetric window function is generated, where the window length corresponds to the samples per chirp (number of lines) and the number of chirps (number of columns).
3. For each transceiver, a (1-dimensional) range Doppler map is calculated based on an average of a succession of radar signals (to reduce noise), windowed by the window function, applying a 2-dimensional Fourier transformation and shifting zero Doppler to the middle of the x axis.
4. Now, the phase difference may be obtained from two range Doppler maps M_{RD,1}, M_{RD,2} by calculating *M*_{*RD,*1} · *̅M̅*̅_̅{̅*̅R̅D̅,̅*̅2̅}̅*.* This step may be repeated or generalized to more than two transceivers.
5. From this product or these products, respectively, the angles of arrival may be calculated from the phases of the matrix elements.

From the range Doppler maps, point clouds are generated by a processing module 31, consisting of all points where a movement was detected by the radar system. Each of the movement points is characterized by its three-dimensional position and its three-dimensional velocity vector. In addition, the multi-dimensional feature vector assigned to the respective point comprises elements relating to the following properties:
- the reflection magnitude;
- the signal-to-noise ratio of the underlying radar measurement(s); and
- a confidence measure obtained from the matrix inversion mentioned above.

The point cloud data is received at regular time intervals ("frames"). Suitable frequencies are e. g. 10 Hz, i. e. a frame is received every 0.1 s.

Now, a dedicated, density-based clustering algorithm is run on a clustering module 33, using unsupervised learning methods to group the movement points of each frame according to their characteristics, based on the information contained in the feature vectors. The clustering algorithm is also configured to eliminate noise by detecting not-clustered points ("outliers") and irregular groups. A cluster is a group of movement points which belong to the same moving object. It can have an arbitrary form and size, depending on the detected movements and their distribution.

Two clustering processes are run in parallel:
- For the clustering of entire people, no further parameters are added to the feature vector, as different body parts can move in different directions and have different levels of reflected energy.
- For the clustering of specific body parts (e.g. chest, extremities) the velocity vector of the movement is added to every point's feature vector. This is important as all the detected movements of a specific part are expected to move in more or less the same direction with more or less the same velocity. This creates a high-dimensional clustering space which results in smaller, more accurate clusters.

In both processes, the signal-to-noise ratio and/or the confidence measure may be employed to suitably weigh the movement points in the analysis, in order to improve the quality of the clustering.

In order to observe a person and its body parts over time, each cluster is tracked from frame to frame by a tracking module 35. The tracking algorithm calculates for every cluster in every frame the probability that it is the same cluster for a previous frame. The probability calculation takes into account different characteristics of the group - current position, path so far (direction and velocity) and points' reflection characteristics. Each tracked group is then identified as an "object" and is given a unique identifier (ID).

In a filtering module 37, the objects are classified to identify non-human objects. The classification is done by a machine-learning algorithm which was trained on a dedicated high-dimensional feature vector extracted from every object. Objects which are classified as non-humans are being removed and their points' data is not directly used for the identification of postures or posture transitions.

In a further step, the remaining, i. e. human objects are classified as static or dynamic. For that purpose, the positions and velocities of these remaining objects are studied during a time interval. If the overall movement is low and/or the position of the object is restricted to a certain location, the object is classified as static, otherwise it is classified as dynamic. Those remaining human objects which are static and located in a pre-defined bed-zone are identified as patients. In contrast, dynamic human objects are considered to be caregivers. This allows inter alia to detect when a patient is being cared for based on the positional relationship between the objects representing a patient and a caregiver.

The following information is obtained and held available for each of the objects:
- the size and form of the respective cluster: this indicates the volume of the person's body or body part which is producing movements;
- the density and distribution of the movement points in the respective cluster: where the density of the movement-points is higher, more movement is being generated; therefore, changes in the distribution indicate the amplitude of movements in certain areas of the cluster, i.e. of different body parts; and
- the velocity and direction of movement for each point in the respective cluster: this gives an indication of the changes in movements in a higher resolution.

Based on this data, the following related information may be inferred:
- the movement-patterns of the person represented by the associated groups;
- the movement-patterns of (some of) his or her body parts;
- the amplitude and frequencies of those movements;
- some information about the environment in which those movements are taking place (e.g. height above floor, distance from other people/objects etc.)

Next, the posture of each of the objects relating to patients is determined. This is done in one of two ways:
1. If the object exhibits substantial movement with generally uniform distribution in the point cloud, the shape thereof represents more or less the shape of the person. The classification of the basic three postures 'lying', 'sitting' or 'standing' is done in a classification module 41, using a machine learning algorithm, trained on labeled samples, based on the entire point cloud representing the patient.
2. If there is less movement, the determination of the posture is based on the tracked groups representing body parts. In order to do so, first the body part group representing the chest is identified in identification module 43. This should always be possible, because the chest of a living person always generates movement.

The detection of the chest is done as follows:
1) The current movement activity level is determined for the respective patient. This activity level may correspond to the variance of the IQ signal summed up for the respective cluster(s). If this value exceeds a certain threshold, the patient is classified by classifier module 42 as presently active, if not, the subject is classified as presently non-active. The threshold may depend on the specific radar system 10 and its arrangement in the hospital room and relative to the beds 2.1...3. Accordingly, the threshold is determined during a calibration process of the system. Automatic recalibration is possible, e. g. during intervals when no patient is present in the monitored area.
   In case of an active classification that is confirmed during a certain first time period (e. g. 2 seconds), the status of the patient is switched to "active" or kept at "active" - in case of such a switch, the past interval according to the first time period is retroactively assigned to active status. If the status is "active" and the classification has been non-active for a certain second time period (e. g. the past 10 seconds) the status is switched to "non-active". Accordingly, every point in time is assigned to an "active" or "non-active" interval, wherein active and non-active intervals follow each other alternately.
2) Each cluster related to the patient's body parts is evaluated according to its geometrical three-dimensional shape. Clusters which their shape and size cannot be representing a chest (e.g. too large, too long) are filtered out.
3) For all the remaining clusters, their movement-points are collected every 125ms and a single value is calculated. This single value takes into account the number of points collected in this time period as well as the absolute velocity of each point. It is a measure for the activity level in the respective cluster.
4) The single values for each cluster are collected over a period of time representing 5-6 breathing cycles in inactive state (ca. 1 minute).
5) A Fast Fourier transform (FFT) is performed over the collected indexes for each cluster to detect the frequencies in the data. The stronger the ~0.07-0.17 Hz frequency is present, the higher the probability is, that the cluster is the person's chest.
6) Now, the object relating to the identified cluster is defined as being the patient's chest.

The other body part groups (representing e. g. the head, the legs or the hands) are then identified by identification module 45 according to their position relative to the chest. The posture is determined through analysis of the position of the recognized body parts (e.g. a movement close and above the chest will suggest that the person is sitting etc.). If only a chest is detected, i. e. if the patient is very still, an analysis of the other groups' cluster's shapes as well as the chest-movement's vector identifies whether the patient is e. g. lying or sitting. The person is continually tracked. If the position of the person changes, the chest-detection process is repeated.

Basically, the steps described above are repeated to have updated information on the situation. The repetition / update frequency for the different phases may be different. The radar measurements are repeated frequently, with a period of 1 s or less. The same applies to the steps leading up to the determination of the activity level.

After the posture has been detected, in a detection module 51 every movement-point which is generated by the represented object is mapped to the posture to detect deviations. If a critical amount of points are located outside of the expected posture, the transition type is detected by analyzing the position of the deviated movements (e.g. if the person was lying and the movements are above the posture, then a sitting transition is assumed etc.).

In addition, the tracker's positions from each object are compared from frame to frame to detect changes in the location in the room without posture change (e.g. moving with a wheelchair, rolling from a floor-bed etc.).

The processes described above may be supported by a low-level processing of the range Doppler data, whereby information on obtained clusters, defined objects and/or identified body parts is used to identify regions of interest in the range Doppler map. These regions of interest may be specifically analyzed in order to:
- identify valid radar measurements to be considered for obtaining the point cloud data;
- select radar measurements to be considered for extracting vital sign information; and
- clustering micro Doppler radar measurements in one of the regions of interest or starting from a region of interest.

The filtering of the radar measurements may be directed to the identification of body parts. For that purpose, radar data relating to events involving speeds outside a certain speed range or accelerations outside a certain acceleration range may be filtered out as this data might be due to artefacts or relate to irrelevant events. Similarly, radar measurements that are relevant for the extraction of vital sign information may be identified at an early stage, filtering out all the rest, thus greatly simplifying the further analysis. The clustering of micro Doppler radar measurements in certain regions facilitates the classification of specific movement patterns or the extraction of movement anomalies.

From the posture and transition data, a probability (either discrete or continuous) that the person will stand up or change his location is calculated in estimation module 61. The calculation is done in one of the following methods:
1. Direct analysis: From healthcare research papers and other knowledge bases known situations which indicate that a person is about to stand-up or change his location are identified. Each of these situations is manually being analyzed to determine what movement patterns it generates. The system is then either programmed or trained to identify those (and similar) patterns. After identified, each situation is mapped to its stand-up probability, as interpreted from the information obtained from the knowledge bases.
2. Statistical analysis: For this method, data recorded from the radar is used, in which every occurrence of a person standing-up or changing his or her location is marked (labeled). Those recordings are fed into a learning system, which then directly recognizes which patterns precede stand-up and location-change occurrences. Given enough recordings, the system calculates for each movement pattern the probability that it will lead to a person standing up or changing his or her location. After the system has processed all those recordings ("training"), it can continuously analyze data from movement-patterns and map them to the learned probabilities. If a pattern is not recognized, the probability "0" is assumed.

Each posture-transition and location-change has a pre-defined value on a scale of fall risk, whereas a lying person has the lowest and a standing/walking person has the highest risk of falling.

The system offers an interface, in which a caregiver can chose for each patient at which stand up probability value or fall risk value he or she would like to be informed. The possible values to choose from are in generally discrete, but theoretically the system could offer a continuous model. This allows individual configuration according to patient and staff needs and so minimize the number of unnecessary alarms (e.g. a person with low risk of falling and/or who is standing up slowly might trigger an alarm only when he or she stands up, while a person with a high risk of falling and/or who is standing up quickly should trigger an alarm already when he or she is sitting on the bed's edge or even before, during a respective transition or if there are strong indications that such a transition is about to happen). This will make sure that the caretaker can arrive at the patient before he or she falls.

As an example, if a person's position in bed changes from lying to sitting, the probability that he or she is about to leave the bed rises dramatically to about 80%. The detection of a sitting-up person can be done in many ways. For example, if the height of the bed and the height of the (ceiling mounted) radar are known, a sit-up detection can be done merely by analyzing the distance of the person from the radar.

If the person lifts his or her head, but generally does not produce any noticeable movements with the rest of his or her body, he is most likely only observing the room and the stand-up probability is low (20%). On the other hand, if at the same time the person's body rotates horizontally around the center of gravity, the stand-up probability rises significantly to about 80% (as the rotation will at a certain stage result in the legs leaving the surface of the bed and reaching the floor).

This means that if a caretaker chooses to be alerted at a stand-up probability of 20%, he or she will most likely be informed earlier than if he choose a probability of 80% (as people usually first look around and then start rotating). But at the same time he or she might receive more false alerts, e.g. when the patients only wants to look around and has no intention of standing-up.

With respect to a person sitting in a chair, if his or her hands are kept close to his body, the stand-up probability is relatively low (10-30%), as, especially for elderly people, the hands are required for assisting in standing up. When a sitting person stretches his or her arms and bends forward, it may indicates that he or she is looking for something to hold on to (e.g. a rolling-walker) and therefore the stand-up probability rises to about 75%.

If the fall risk exceeds the patient-specific threshold, the caregiver is alerted by an alerting module 71, connected to an interface 73. The interface 73 may connect the alerting module 71 or, more precisely, a server running the alerting module 71 to a wired or wireless network, such as a LAN or WLAN network, and allow for forwarding alerts to personal devices of the caregivers or to alerting equipment of the hospital.

Beside fall prevention the caregivers may be informed when a person changes his or her location, even if not directly associated with the prevention of a fall. Those cases include rolling off a floor-bed, moving (out of the room) with a wheel chair etc.

It is to be noted that the modules and processors may be realized by software running on a server, a general purpose or dedicated computer. Some or all of the modules may be bound to dedicated hardware units. Similarly, some of the modules may be part of the radar system, whereas other modules are part of external components. It may be advantageous to run some of the modules on a cloud server to reduce the required local computing power and/or to centralize data storage.

The invention is not restricted to the described embodiments. As an example, instead of a MIMO UWB radar system usual phased arrays may be used and/or wideband radars having smaller bandwidths such as about 150 MHz.

The frequency of the radar system may be adjusted to the purpose of the system. As an example, higher frequencies of up to 120 GHz or more may be used as long as the required penetration capacity is achieved.

In three-dimensional systems, more than three receiving antennas may be employed. In two-dimensional systems, two antennas may be sufficient. In principle, there may be a single transmitting antenna or a plurality of transmitting antennas. Furthermore, as mentioned above a plurality of antennas may be replaced by a moving (e. g. rotating) antenna that effectively provides multiple receiving (and/or sending) locations.

The embodiment related to a hospital environment. The inventive method and system may be employed in other environments, including nursing homes and the monitored people's homes.

In summary, it is to be noted that the invention creates a method for determining a posture of a human being that allows for a reliable determination based on a limited number of sensor modalities.

## Claims

1. A method for determining a posture of a human being, comprising the following steps:
a) obtaining measurement data from at least two dimensional radar measurements;
b) processing the measurement data to obtain at least two dimensional point cloud data, in particular three dimensional point cloud data, a position and local data being assigned to each of the points of the point cloud data, the local data comprising velocity vectors of the points, wherein the point cloud data is received at regular time intervals corresponding to frames;
c) clustering the point cloud data to obtain clustered data representing the human being by a plurality of clusters, wherein a cluster is a group of points of the point cloud which belongs to the same moving object;
d) tracking the clusters from frame to frame over time to define a plurality of objects, wherein
- it is calculated for every cluster in every frame the probabilities that it relates to the same detected object as a certain cluster in the previous frame,
- for the clustering of body parts the velocity vectors of the points of the point cloud are taken into account,
- for defining the objects data obtained from tracking the clusters, covering a time interval, is analyzed to separate static from dynamic objects;
e) identify body parts of the human being and positional relationships among them based on the plurality of objects, wherein the identified body parts comprise the head and/or limbs; and
f) analyze the positional relationships to determine the posture of the human being.

2. The method as recited in claim 1, **characterized in that** the local data assigned to each of the points of the point cloud data comprises a reflection magnitude.

3. The method as recited in any of claims 1 or 2, **characterized in that** the local data assigned to each of the points of the point cloud data comprises a signal-to-noise ratio of an underlying radar measurement and/or a measure for a confidence of the position and/or the local data.

4. The method as recited in any of claims 1 to 3, **characterized in that** the objects are classified to identify non-human objects and **in that** objects representing non-human objects are excluded from the identification of the body parts of the human being and the positional relationships among them.

5. The method as recited in claim 1, **characterized in that** static objects located in predefined zones are identified as patients.

6. The method as recited in any of claims 1 to 5, **characterized in that** an activity level is determined from the point cloud data and **in that** an overall shape defined by the points of the plurality of clusters representing the human being is analyzed to determine the posture if the activity level exceeds a threshold.

7. The method as recited in any of claims 1 to 6, **characterized by** the further steps of
- mapping further points of the point cloud data, obtained from further measurements after determining the posture, to the determined posture;
- identifying points of the mapped further points lying outside of the determined posture in order to identify a posture transition.

8. The method as recited in any of claims 1 to 7, **characterized in that** a risk for the human being experiencing a fall incident is determined based on the posture and/or the posture transition and **in that** an alarm condition is set for the human being if the risk exceeds a threshold.

9. The method as recited in claim 8, **characterized in that** the alarm condition for the human being is reset if it is determined that the human being is approached by a dynamic object representing a caregiver.

10. The method as recited in any of claims 1 to 9, **characterized in that** a range Doppler map is obtained from the at least two dimensional radar measurements and **in that** information on obtained clusters, defined objects and/or identified body parts is used to identify regions of interest in the range Doppler map, the regions of interest of the range Doppler map being analyzed in order to do at least one of the following:
- identify valid radar measurements to be considered for obtaining the at least two dimensional point cloud data;
- select radar measurements to be considered for extracting vital sign information; and
- clustering micro Doppler radar measurements in one of the regions of interest or starting from a region of interest.

11. The method as recited in any of claims 1 to 10, **characterized by** the further steps of identifying an object that represents a chest of a human being, based on characteristic features relating to breathing movements and/or heartbeat; and determining a positional relationship of other objects to the identified chest object to identify the body parts of the human being and the positional relationships among them.

12. The method as recited in claim 11, **characterized in that** a movement activity level is determined from an amplitude and/or phase of the measurement data, that the activity level is classified into at least one high activity level class and into at least one low activity level class and **in that** the amplitude and/or phase of the measurement data relating to an activity level classified in the at least one low activity level class is analyzed to identify the position of the chest region.

13. The method as recited in claim 11 or 12, **characterized in that** each object is classified according to its geometrical shape into a torso candidate class or a non-torso candidate class.

14. The method as recited in any of claims 11 to 13, **characterized in that** for each of the objects a single value is calculated for all the points of the point cloud assigned to the respective cluster, collected during a first sample period, the single value representing an activity level in the respective cluster.

15. The method as recited in claim 14, **characterized in that** the single value is calculated from a number of points collected and absolute velocities of each of the points.

16. The method as recited in claim 14 or 15, **characterized in that** the single values are collected over a second sample period.

17. The method as recited in claim 16, **characterized by** performing a frequency analysis over the collected single values and classifying each of the objects into a chest candidate class or a non-chest candidate class.

18. System for determining a posture of a human being, comprising:
a) a radar system for generating measurement data from at least two dimensional radar measurements from a space accommodating the human being;
b) a first processor adapted to process the measurement data to obtain at least two dimensional point cloud data, in particular three dimensional point cloud data, a position and local data being assigned to each of the points of the point cloud data, the local data comprising velocity vectors of the points, wherein the point cloud data is received at regular time intervals corresponding to frames;
c) a second processor adapted to cluster the point cloud data to obtain clustered data representing the human by a plurality of clusters; wherein a cluster is a group of points of the point cloud which belongs to the same moving object;
d) a tracker adapted to track the clusters from frame to frame over time to define objects, wherein the tracker is adapted to
- calculate for every cluster in every frame the probabilities that it relates to the same detected object as a certain cluster in the previous frame;taking into account the velocity vectors of the points of the point cloud for the clustering of body parts;
- analyse data obtained from tracking the clusters, covering a time interval, to separate static from dynamic objects, for defining the objects;
e) an identifier adapted to identify body parts of the human being and positional relationships among them based on the plurality of objects, wherein the identified body parts comprise the head and/or limbs; and
f) an analyzer adapted to analyze the positional relationships to determine the posture of the human being.

## Patentansprüche

1. Verfahren zur Bestimmung einer Körperhaltung eines Menschen, umfassend die folgenden Schritte:
a) Erhalten von Messdaten aus mindestens zweidimensionalen Radarmessungen;
b) Verarbeiten der Messdaten, um mindestens zweidimensionale Punktwolkendaten, insbesondere dreidimensionale Punktwolkendaten, zu erhalten, wobei jedem der Punkte der Punktwolkendaten eine Position und lokale Daten zugewiesen werden, wobei die lokalen Daten Geschwindigkeitsvektoren der Punkte umfassen, wobei die Punktwolkendaten in regelmäßigen Zeitintervallen entsprechend Frames empfangen werden;
c) Clustern der Punktwolkendaten, um geclusterte Daten zu erhalten, die das menschliche Wesen durch eine Vielzahl von Clustern repräsentieren, wobei ein Cluster eine Gruppe von Punkten der Punktwolke ist, die zu demselben sich bewegenden Objekt gehört;
d) Verfolgen der Cluster von Frame zu Frame über die Zeit, um eine Vielzahl von Objekten zu definieren, wobei
- für jedes Cluster in jedem Frame die Wahrscheinlichkeiten berechnet werden, dass es sich auf dasselbe detektierte Objekt wie ein bestimmtes Cluster in dem vorherigen Frame bezieht,
- für das Clustern von Körperteilen die Geschwindigkeitsvektoren der Punkte der Punktwolke berücksichtigt werden,
- zum Definieren der Objekte Daten, die aus dem Verfolgen der Cluster erhalten werden und die ein Zeitintervall abdecken, analysiert werden, um statische von dynamischen Objekten zu trennen;
e) Identifizieren von Körperteilen des Menschen und von Positionsbeziehungen zwischen ihnen basierend auf der Vielzahl von Objekten, wobei die identifizierten Körperteile den Kopf und/oder die Gliedmaßen umfassen; und
f) Analysieren der Positionsbeziehungen, um die Körperhaltung des Menschen zu bestimmen.

2. Verfahren nach Anspruch 1, **dadurch gekennzeichnet, dass** die lokalen Daten, die jedem der Punkte der Punktwolkendaten zugeordnet sind, eine Reflexionsgröße umfassen.

3. Verfahren nach einem der Ansprüche 1 oder 2, **dadurch gekennzeichnet, dass** die lokalen Daten, die jedem der Punkte der Punktwolkendaten zugeordnet sind, ein Signal-RauschVerhältnis einer zugrundeliegenden Radarmessung und/oder ein Maß für eine Konfidenz der Position und/oder der lokalen Daten umfassen.

4. Verfahren nach einem der Ansprüche 1 bis 3, **dadurch gekennzeichnet, dass** die Objekte klassifiziert werden, um nichtmenschliche Objekte zu identifizieren, und dass Objekte, die nichtmenschliche Objekte repräsentieren, von der Identifizierung der Körperteile des Menschen und der Positionsbeziehungen zwischen ihnen ausgeschlossen werden.

5. Verfahren nach Anspruch 1, **dadurch gekennzeichnet, dass** statische Objekte, die sich in vordefinierten Zonen befinden, als Patienten identifiziert werden.

6. Verfahren nach einem der Ansprüche 1 bis 5, **dadurch gekennzeichnet, dass** ein Aktivitätsniveau aus den Punktwolkendaten bestimmt wird und dass eine Gesamtform, die durch die Punkte der mehreren Cluster, die den Menschen repräsentieren, definiert wird, analysiert wird, um die Körperhaltung zu bestimmen, wenn das Aktivitätsniveau einen Schwellenwert überschreitet.

7. Verfahren nach einem der Ansprüche 1 bis 6, **gekennzeichnet durch** die folgenden weiteren Schritte:
- Abbilden weiterer Punkte der Punktwolkendaten, die aus weiteren Messungen nach dem Bestimmen der Körperhaltung erhalten werden, auf die bestimmte Körperhaltung;
- Identifizieren von Punkten der abgebildeten weiteren Punkte, die außerhalb der bestimmten Körperhaltung liegen, um einen Körperhaltungsübergang zu identifizieren.

8. Verfahren nach einem der Ansprüche 1 bis 7, **dadurch gekennzeichnet, dass** ein Risiko für den Menschen, einen Sturzvorfall zu erfahren, basierend auf der Körperhaltung und/oder dem Körperhaltungsübergang bestimmt wird und dass eine Alarmbedingung für den Menschen eingestellt wird, wenn das Risiko einen Schwellenwert überschreitet.

9. Verfahren nach Anspruch 8, **dadurch gekennzeichnet, dass** die Alarmbedingung für den Menschen zurückgesetzt wird, wenn bestimmt wird, dass der Mensch von einem dynamischen Objekt, das eine Pflegeperson repräsentiert, angenähert wird.

10. Verfahren nach einem der Ansprüche 1 bis 9, **dadurch gekennzeichnet, dass** eine Range-Doppler-Karte aus den mindestens zweidimensionalen Radarmessungen erhalten wird und dass Informationen über erhaltene Cluster, definierte Objekte und/oder identifizierte Körperteile verwendet werden, um Regionen von Interesse in der Range-Doppler-Karte zu identifizieren, wobei die Regionen von Interesse der Range-Doppler-Karte analysiert werden, um mindestens eines der Folgenden zu tun:
- Identifizieren gültiger Radarmessungen, die zum Erhalten der mindestens zweidimensionalen Punktwolkendaten zu berücksichtigen sind;
- Auswählen von Radarmessungen, die zum Extrahieren von Vitalzeicheninformationen zu berücksichtigen sind; und
- Clustern von Mikro-Doppler-Radarmessungen in einer der Regionen von Interesse oder ausgehend von einer Region von Interesse.

11. Verfahren nach einem der Ansprüche 1 bis 10, **gekennzeichnet durch** die folgenden weiteren Schritte: Identifizieren eines Objekts, das eine Brust eines Menschen repräsentiert, basierend auf charakteristischen Merkmalen, die sich auf Atembewegungen und/oder Herzschlag beziehen; und Bestimmen einer Positionsbeziehung anderer Objekte zu dem identifizierten Brustobjekt, um die Körperteile des Menschen und die Positionsbeziehungen zwischen ihnen zu identifizieren.

12. Verfahren nach Anspruch 11, **dadurch gekennzeichnet, dass** ein Bewegungsaktivitätsniveau aus einer Amplitude und/oder Phase der Messdaten bestimmt wird, dass das Aktivitätsniveau in mindestens eine Klasse mit hohem Aktivitätsniveau und in mindestens eine Klasse mit niedrigem Aktivitätsniveau klassifiziert wird und dass die Amplitude und/oder Phase der Messdaten, die sich auf ein Aktivitätsniveau beziehen, das in der mindestens einen Klasse mit niedrigem Aktivitätsniveau klassifiziert ist, analysiert wird, um die Position der Brustregion zu identifizieren.

13. Verfahren nach Anspruch 11 oder 12, **dadurch gekennzeichnet, dass** jedes Objekt gemäß seiner geometrischen Form in eine Torsokandidatenklasse oder eine Nicht-Torsokandidatenklasse klassifiziert wird.

14. Verfahren nach einem der Ansprüche 11 bis 13, **dadurch gekennzeichnet, dass** für jedes der Objekte ein einziger Wert für alle Punkte der Punktwolke, die dem jeweiligen Cluster zugeordnet sind, die während einer ersten Abtastperiode gesammelt werden, berechnet wird, wobei der einzige Wert ein Aktivitätsniveau in dem jeweiligen Cluster repräsentiert.

15. Verfahren nach Anspruch 14, **dadurch gekennzeichnet, dass** der einzige Wert aus einer Anzahl von gesammelten Punkten und absoluten Geschwindigkeiten jedes der Punkte berechnet wird.

16. Verfahren nach Anspruch 14 oder 15, **dadurch gekennzeichnet, dass** die einzigen Werte über eine zweite Abtastperiode gesammelt werden.

17. Verfahren nach Anspruch 16, **gekennzeichnet durch** Durchführen einer Frequenzanalyse über die gesammelten einzigen Werte und Klassifizieren jedes der Objekte in eine Brustkandidatenklasse oder eine Nicht-Brustkandidatenklasse.

18. System zur Bestimmung einer Körperhaltung eines Menschen, umfassend:
a) ein Radarsystem zum Erzeugen von Messdaten aus mindestens zweidimensionalen Radarmessungen aus einem Raum, in dem sich der Mensch befindet;
b) einen ersten Prozessor, der dazu ausgelegt ist, die Messdaten zu verarbeiten, um mindestens zweidimensionale Punktwolkendaten, insbesondere dreidimensionale Punktwolkendaten, zu erhalten, wobei jedem der Punkte der Punktwolkendaten eine Position und lokale Daten zugewiesen werden, wobei die lokalen Daten Geschwindigkeitsvektoren der Punkte umfassen, wobei die Punktwolkendaten in regelmäßigen Zeitintervallen entsprechend Frames empfangen werden;
c) einen zweiten Prozessor, der dazu ausgelegt ist, die Punktwolkendaten zu clustern, um geclusterte Daten zu erhalten, die den Menschen durch eine Vielzahl von Clustern repräsentieren; wobei ein Cluster eine Gruppe von Punkten der Punktwolke ist, die zu demselben sich bewegenden Objekt gehört;
d) einen Tracker, der dazu ausgelegt ist, die Cluster von Frame zu Frame über die Zeit zu verfolgen, um Objekte zu definieren, wobei der Tracker dazu ausgelegt ist,
- für jedes Cluster in jedem Frame die Wahrscheinlichkeiten zu berechnen, dass es sich auf dasselbe detektierte Objekt wie ein bestimmtes Cluster in dem vorherigen Frame bezieht; unter Berücksichtigung der Geschwindigkeitsvektoren der Punkte der Punktwolke für das Clustern von Körperteilen;
- Daten, die aus dem Verfolgen der Cluster erhalten werden und die ein Zeitintervall abdecken, zu analysieren, um statische von dynamischen Objekten zu trennen, um die Objekte zu definieren;
e) einen Identifizierer, der dazu ausgelegt ist, Körperteile des Menschen und Positionsbeziehungen zwischen ihnen basierend auf der Vielzahl von Objekten zu identifizieren, wobei die identifizierten Körperteile den Kopf und/oder die Gliedmaßen umfassen; und
f) einen Analysator, der dazu ausgelegt ist, die Positionsbeziehungen zu analysieren, um die Körperhaltung des Menschen zu bestimmen.

## Revendications

1. Procédé pour déterminer la posture d'un être humain, comprenant les étapes suivantes consistant à :
a) obtenir des données de mesure à partir de mesures radar au moins bidimensionnelles ;
b) traiter les données de mesure pour obtenir des données de nuage de points bidimensionnelles, notamment des données de nuage de points tridimensionnelles, une position et des données locales étant assignées à chacun des points des données de nuage de points, les données locales comprenant des vecteurs de vitesse des points, dans lequel les données de nuage de points sont reçues à des intervalles de temps réguliers correspondant à des trames ;
c) regrouper les données de nuage de points pour obtenir des données regroupées représentant l'être humain sous la forme d'une pluralité de clusters, dans lequel un cluster est un groupe de points du nuage de points qui appartient à un même objet en mouvement ;
d) suivre les clusters d'une trame à une autre trame au fil du temps afin de définir une pluralité d'objets, dans lequel :
- pour chaque cluster dans chaque trame, on calcule les probabilités qu'il corresponde au même objet détecté qu'à un certain cluster dans la trame précédente ;
- pour le regroupement des parties du corps, les vecteurs de vitesse des points du nuage de points sont pris en compte ;
- pour définir les objets, les données issues du suivi des clusters, couvrant un intervalle de temps, sont analysées afin de distinguer les objets statiques des objets dynamiques ;
e) identifier des parties du corps de l'être humain ainsi que les relations de position entre celles-ci, sur la base de la pluralité d'objets, dans lequel les parties du corps identifiées comprennent la tête et/ou les membres ; et
f) analyser les relations de position pour déterminer la posture de l'être humain.

2. Procédé selon la revendication 1, **caractérisé en ce que** les données locales assignées à chacun des points des données de nuage de points comprennent une amplitude de réflexion.

3. Procédé selon une quelconque des revendications 1 ou 2, **caractérisé en ce que** les données locales assignées à chacun des points des données de nuage de points comprennent un rapport signal sur bruit d'une mesure radar sous-jacente et/ou une mesure de confiance concernant la position et/ou les données locales.

4. Procédé selon une quelconque des revendications 1 à 3, **caractérisé en ce que** les objets sont classés afin d'identifier des objets non humains et **en ce que** les objets représentant des objets non humains sont exclus de l'identification des parties du corps de l'être humain et des relations de position entre celles-ci.

5. Procédé selon la revendication 1, **caractérisé en ce que** des objets statiques situés dans des zones prédéfinies sont identifiés comme étant des patients.

6. Procédé selon une quelconque des revendications 1 à 5, **caractérisé en ce qu'**un niveau d'activité est déterminé à partir des données de nuage de points et **en ce qu'**une forme globale, définie par les points de la pluralité de clusters représentant l'être humain, est analysée pour déterminer la posture si le niveau d'activité dépasse un seuil.

7. Procédé selon une quelconque des revendications 1 à 6, **caractérisé par** les étapes supplémentaires consistant à :
- mettre en correspondance des points supplémentaires, issus de données de nuage de points obtenues à partir de mesures effectuées après la détermination de la posture, à la posture déterminée ;
- identifier, parmi les points supplémentaires mis en correspondance, ceux qui se situent en dehors de la posture déterminée, afin d'identifier une transition de posture.

8. Procédé selon une quelconque des revendications 1 à 7, **caractérisé en ce qu'**un risque de chute pour l'être humain est déterminé sur la base de la posture et/ou de la transition de posture et **en ce qu'**une condition d'alarme est définie pour l'être humain si le risque dépasse un seuil.

9. Procédé selon la revendication 8, **caractérisé en ce que** la condition d'alarme concernant l'être humain est réinitialisée si il est déterminé qu'un objet dynamique, représentant un soignant, s'approche de l'être humain.

10. Procédé selon une quelconque des revendications 1 à 9, **caractérisé en ce qu'**une carte de portée radar Doppler est obtenue à partir des mesures radar au moins bidimensionnelles et **en ce que** des informations concernant des clusters obtenus, des objets définis et/ou des parties du corps identifiées sont utilisées pour identifier des régions d'intérêt dans la carte de portée radar Doppler, les régions d'intérêt de la carte de portée radar Doppler étant analysées afin d'effectuer au moins une des opérations suivantes :
- identifier des mesures radar valides à prendre en compte pour obtenir les données de nuage de points au moins bidimensionnelles ;
- sélectionner des mesures radar à prendre en compte pour extraire des informations relatives aux signes vitaux ; et
- regrouper des mesures radar micro-Doppler situées dans une des régions d'intérêt ou à partir d'une région d'intérêt.

11. Procédé selon une quelconque des revendications 1 à 10, **caractérisé en ce qu'**il comprend les étapes supplémentaires consistant à identifier un objet qui représente une poitrine d'un être humain, sur la base des caractéristiques liées aux mouvements respiratoires et/ou aux battements cardiaques ; et à déterminer une relation de position d'autres objets par rapport à l'objet identifié comme étant la poitrine, afin d'identifier les parties du corps de l'être humain ainsi que les relations de position entre celles-ci.

12. Procédé selon la revendication 11, **caractérisé en ce qu'**un niveau d'activité de mouvement es déterminé à partir d'une amplitude et/ou d'une phase des données de mesure, que le niveau d'activité est classé en au moins une classe de niveau d'activité élevé et en au moins une classe de niveau d'activité faible et **en ce que** l'amplitude et/ou la phase des données de mesure relatives à un niveau d'activité classé en au moins une classe de niveau d'activité faible sont analysées afin d'identifier la position de la région thoracique.

13. Procédé selon la revendication 11 ou 12, **caractérisé en ce que** chaque objet est classé, en fonction de sa forme géométrique, en une classe de candidats « torse » ou en une classe de candidats « non-torse ».

14. Procédé selon une quelconque des revendications 11 à 13, **caractérisé en ce que**, pour chacun des objets, une valeur unique est calculée pour l'ensemble des points du nuage de points assignés au cluster correspondant, collectés pendant une première période d'échantillonnage, la valeur unique représentant un niveau d'activité dans le cluster correspondant.

15. Procédé selon la revendication 14, **caractérisé en ce que** la valeur unique est calculée à partir d'un nombre de points collectés et des vitesses absolues de chacun des points.

16. Procédé selon la revendication 14 ou 15, **caractérisé en ce que** les valeurs uniques sont collectées sur une deuxième période d'échantillonnage.

17. Procédé selon la revendication 16, **caractérisé en ce qu'**une analyse fréquentielle est effectuée sur les valeurs uniques collectées et **en ce que** chaque objet est classé en une classe de candidats « thorax » ou en une classe de candidats « non-thorax ».

18. Système de détermination de la posture d'un être humain, comprenant :
a) un système radar pour générer des données de mesure à partir de mesures radar au moins bidimensionnelles effectuées dans un espace dans lequel se trouve l'être humain ;
b) un premier processeur adapté pour traiter les données de mesure afin d'obtenir des données de nuage de points au moins bidimensionnelles, notamment des données de nuage de points tridimensionnelles, une position et des données locales étant assignées à chacun des données de nuage de points, les données locales comprenant des vecteurs de vitesse des points, dans lequel les données de nuage de points sont reçues à des intervalles temporels réguliers correspondant à des trames ;
c) un deuxième processeur adapté pour regrouper les données de nuage de points en clusters, afin d'obtenir des données de cluster représentant l'être humain au moyen d'une pluralité de clusters ; dans lequel un cluster est un groupe de points du nuage de points qui appartient à un même objet en mouvement ;
d) un module de suivi adapté pour suivre les clusters d'une trame à une autre trame au fil du temps afin de définir des objets, dans lequel le module de suivi est configuré pour :
- calculer, pour chaque cluster dans chaque trame, les probabilités qu'il corresponde au même objet détecté qu'un certain cluster dans la trame précédente ; en tenant compte des vecteurs de vitesse des points du nuage de points pour le regroupement des parties du corps ;
- analyser les données issues du suivi des clusters, sur un intervalle de temps donné, afin de distinguer les objets statiques des objets dynamiques, en vue de définir les objets ;
e) un module d'identification adapté pour identifier des parties du corps de l'être humain ainsi que les relations de position entre celles-ci, sur la base de la pluralité d'objets, dans lequel les parties du corps identifiées comprennent la tête et/ou les membres ; et
f) un module d'analyse adapté pour analyser les relations de position afin de déterminer la posture de l'être humain.
